(19) Europäisches Patentamt
European Patent Office
Office européen des brevets

(11) Veröffentlichungsnummer: **0 059 156**
**B1**

# (12) EUROPÄISCHE PATENTSCHRIFT

(45) Veröffentlichungstag der Patentschrift: **23.04.86**

(21) Anmeldenummer: **82730012.0**

(22) Anmeldetag: **12.02.82**

(51) Int. Cl.[4]: **C 07 D 233/64,** C 07 D 401/04, C 07 D 401/12, C 07 D 403/04, C 07 D 403/12

## (54) Verfahren zur Herstellung von Imidazolessigsäurederivaten.

(30) Priorität: **13.02.81 DE 3106150**

(43) Veröffentlichungstag der Anmeldung: **01.09.82 Patentblatt 82/35**

(45) Bekanntmachung des Hinweises auf die Patenterteilung: **23.04.86 Patentblatt 86/17**

(84) Benannte Vertragsstaaten: **AT BE CH DE FR GB IT LI LU NL SE**

(56) Entgegenhaltungen:
**EP - A - 0 005 528**
**CH - A - 524 617**

**CHEMICAL ABSTRACTS, Band 84, Nr. 1, 5. Januar 1976, Seite 423, Nr. 4955x, Columbus, Ohio, USA**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Vorbrüggen, Helmut, Dr. Prof., Wilkestrasse 7, D-1000 Berlin 27 (DE)**
Erfinder: **Schwarz, Norbert, Dr., Stubenrauchstrasse 31, D-1000 Berlin 41 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Diese Erfindung betrifft ein Verfahren zur Herstellung von Imidazolessigsäurederivaten aus 4-Halogenacetessigsäurederivaten und Amidinen.

Imidazol-4(5)-essigsäuren, ihre Ester oder Amide waren bisher nur mit grossem Aufwand über mehrere Stufen herzustellen. Zusätzlich musste bereits in der ersten Stufe im Autoklaven unter Druck gearbeitet werden. Von W. Schunack, [Archiv Pharmazie 307, 470 (1974)], F.L. Pyman, [J. Chem. Soc. 99, 668 (1911)] und in der Europäischen Patentanmeldung 5528 werden mehrstufige Verfahren beschrieben. Wie aus dem Schweizerischen Patent 524 617 zu sehen ist, führt der an sich naheliegende Weg, die leicht zugänglichen 4-Halogenacetessigsäureester oder -amide mit Amidinen umzusetzen, stets zur 6-Ringbildung (6-Halogenmethylpyrimidine), weil das Halogen in diesen α-Halogenketonen nicht reaktiv genug ist.

Es wurde nun gefunden, dass 4-Halogenacetessigsäureester ganz anders mit Amidinen reagieren, wenn man die Acetessigsäurederivate zuerst in 4-Halogen-3-trialkylsilyloxy- bzw. -3-alkoxycrotonsäureester umwandelt. Das 4-Halogen reagiert nun überraschenderweise sehr leicht und die 4-Halogenacetessigsäurederivate setzen sich mit Amidinen zu 1,2-substituierten Imidazol-4(5)-essigsäureestern um.

Gegenstand der Erfindung ist ein Verfahren zur Herstellung von Imidazolessigsäurederivaten der allgemeinen Formel I,

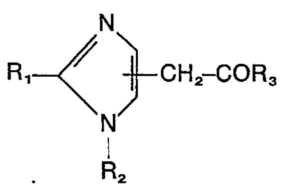

(I),

in der

$R_1$ Wasserstoff, eine geradkettige oder verzweigte Alkylgruppe mit 1–24 C-Atomen, Cyclopentyl, Cyclohexyl, Cycloheptyl, Äthylcyclopentyl, Methylcyclohexyl, einen Phenylalkylrest mit 7–10 C-Atomen, die Reste Phenyl, α-Naphthyl, β-Naphthyl und Phenanthryl, die durch Chlor, Brom, Methyl, Methoxy oder Trifluormethyl substituiert sein können, eine mono- oder bicyclische heterocyclische Gruppe mit 1–3 N-, O- oder S-Heteroatomen,

$R_2$ Wasserstoff, eine geradkettige oder verzweigte Alkylgruppe mit 1–24 C-Atomen, Cyclopentyl, Cyclohexyl, Cycloheptyl, Äthylcyclopentyl, Methylcyclohexyl, einen Phenylalkylrest mit 7–10 C-Atomen, die Reste Phenyl, α-Naphthyl, β-Naphthyl und Phenanthryl, die durch Chlor, Brom, Methyl, Methoxy oder Trifluormethyl substituiert sein können, eine mono- oder bicyclische heterocyclische Gruppe mit 1–3 N-, O- oder S-Heteroatomen,

$R_1$ und $R_2$ gemeinsam eine Alkylengruppe mit 4–7 C-Atomen und

$R_3$ eine geradkettige oder verzweigte Alkoxygruppe mit 1–10 C-Atomen oder eine Phenylalkoxygruppe mit 7–10 C-Atomen bedeuten, dadurch gekennzeichnet, dass man Amidine bzw. deren Salze der allgemeinen Formel II,

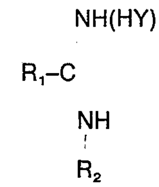

(II),

in der $R_1$ und $R_2$ die oben angegebenen Bedeutungen haben und HY anorganische Mineralsäuren oder organische Säuren darstellt, mit Acetessigsäurederivaten der allgemeinen Formel III,

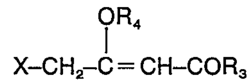

(III),

in der $R_3$ die oben angegebene Bedeutung hat und X die Halogene Fluor, Chlor, Brom und Jod und $R_4$ einen Tri-($C_1$–$C_4$)-alkylsilylrest oder einen $C_1$–$C_4$-Alkylrest darstellen, umsetzt.

Mit $R_1$ und $R_2$ in der Bedeutung einer Alkylgruppe sind geradkettige und verzweigte gesättigte Kohlenwasserstoffreste mit 1–24 C-Atomen, wie z.B. Methyl, Äthyl, Propyl, Isopropyl, Butyl, Isobutyl, sek.-Butyl, tert.-Butyl, Pentyl, Isopentyl, Hexyl, Isohexyl, Heptyl, Oktyl, Nonyl, Decyl, insbesondere solche mit 1–6 C-Atomen gemeint.

Als Phenylalkylreste $R_1$ und $R_2$ sind geradkettige und verzweigte Reste mit 7–10 C-Atomen zu verstehen, wie z.B. Benzyl, 1-Phenyläthyl, 2-Phenyläthyl, 1-Phenylpropyl, 2-Phenylpropyl, 3-Phenylpropyl, 4-Phenylbutyl.

Als heterocyclische Gruppe für $R_1$ und $R_2$ kommen mono- oder bicyclische Heterocyclen mit 1–3 N-, O- oder S-Heteroatomen in Betracht: Pyrryl, 2- oder 3-Thienyl, 2-, 3- oder 4-Pyridyl, Imidazolyl, Pyrazolyl, Triazolyl, Oxazolyl, 2-Thiazolyl, Thiadiazolyl, Oxadiazolyl, Pyrimidyl, Pyrazinyl, Pyridazinyl, Azepinyl, 1, 3 oder 4-Isochinolyl, 2-, 3- oder 4-Chinolyl.

Die aromatischen bzw. heteroaromatischen Reste können die Halogenatome Cl und Br, Methyl, Methoxy oder $CF_3$ enthalten.

Wenn $R_1$ und $R_2$ gemeinsam einen Ring bilden, so ist damit eine Alkylenbrücke gemeint: Tetramethylen, Pentamethylen, Hexamethylen, Heptamethylen.

Die Alkylreste in den Alkoxyresten von $R_3$ sind geradkettige und verzweigte gesättigte Kohlenwasserstoffreste mit 1–10 C-Atomen, wie sie für die Alkylreste $R_1$ und $R_2$ schon benannt worden sind. Das gleiche trifft für die Phenylalkylreste in den Phenylalkoxyresten von $R_3$ zu. Sie entsprechen ebenfalls den für $R_1$ und $R_2$ genannten Resten. Die Heteroarylreste in der Heteroarylaminogruppe von $R_3$ sind identisch mit den für $R_1$ und $R_2$ genannten heterocyclischen Resten.

Die Reaktion der Amidine oder ihrer Salze der allgemeinen Formel (II) mit den entsprechenden O-silylierten bzw. O-alkylierten 4-Halogencrotonsäureestern der allgemeinen Formel (III) wird zweckmässigerweise in einem polaren inerten Lösungsmittel wie Acetonitril, Dimethylformamid (DMF), Dimethylacetamid, N-Methylpyrrolidon, Sulfolan bzw. ohne Lösungsmittel, z.B. in Gegenwart von überschüssigem Hexamethyldisilazan (HMDS) bzw. Trimethylchlorsilan (TCS), bevorzugt beim Siedepunkt des Reaktionsgemisches durchgeführt. Typische Reaktionszeiten sind 0,5–100 Stunden. Das Verhältnis der Reaktanten III und II ist allgemein 1–10, bevorzugt 1–6. Gewöhnlich werden aber stöchiometrische Mengen benutzt.

Ein weiterer Vorteil des neuen Verfahrens liegt darin, dass die Reaktion ausgehend vom 4-Halogenacetessigsäureester in einem Schritt zu den gewünschten Imidazol-4(5)-essigsäureestern führt. Die 4-Halogen-3-silyloxy (vorzugsweise trimethylsilyloxy)crotonsäureester der allgemeinen Formel III werden dazu zweckmässigerweise in situ aus den entsprechenden 4-Halogenacetessigsäureestern mittels Hexamethyldisilazan (HMDS) bzw. der Kombination HMDS und Trimethylchlorsilan (TCS) [S.H. Langer et al., J.O.C. 23, 50 (1958)] dargestellt, was die Imidazolsynthese zu einer Einstufensynthese vereinfacht.

Oft ist es auch zweckmässig, der Reaktion zusätzlich zur Base Hexamethyldisilazan (HMDS) noch eine tertiäre Base wie Triäthylamin, Tripropylamin, Tributylamin, Diisopropyläthylamin bzw. Diazabicyclononen (DBN) oder Diazabicycloundecen (DBU) zuzusetzen.

Die Natur der Säure HY in Verbindungen der Formel II ist völlig unkritisch. Als Mineralsäuren sind geeignet: Chlorwasserstoff, Bromwasserstoff, Schwefelsäure, Salpetersäure, Phosphorsäure. Als organische Säuren kommen in Betracht $C_{1-10}$-Mono-, Di- oder Tricarbonsäuren wie Essigsäure, Oxalsäure, Zitronensäure, Bernsteinsäure, Buttersäure, Maleinsäure, Glutarsäure, Propionsäure, Malonsäure, Sebacinsäure usw. Um die Reaktion zu beschleunigen bzw. die Reaktionstemperatur zu erhöhen, ist es oft von Vorteil, das bei der Kondensation entstehende Hexamethyldisiloxan ($(CH_3)_3Si{-}O{-}Si(CH_3)_3$ Siedepunkt 99°) aus dem Reaktionsgemisch über eine kurze Destillationskolonne destillativ zu entfernen.

Die 4-halogenierten 3-Alkoxycrotonsäureester der Formel (III) lassen sich nach Literaturangaben entweder durch O-Alkylierung von 4-Halogenacetessigsäureestern z.B. mit Orthoameisensäureestern [vgl. U. Schmidt et al., Monatshefte der Chemie 102, 214 (1971)] bzw. durch Halogenierung der entsprechenden 3-Alkoxycrotonsäureester z.B. mit NBS in $CCl_4$ darstellen [vgl. D.G.F.R. Kostermans, Rec. 70, 79 (1951), E.B. Reid and W.R. Ruby, JACS 73, 1054 (1951)].

Die Darstellung der Ausgangsamidine bzw. ihrer Salze der Formel (II) erfolgt nach den in der Literatur, z.B. Houben/Weyl 11/2, S. 38–69 (1958), «The Chemistry of Amidines and Imidates» in «The Chemistry of Functional Groups», Editor Saul Patai, John Wiley and Sons (1975), angegebenen Methoden, vorzugsweise jedoch durch Überführung entsprechend substituierter Nitrile in die zugehörigen Imidoester-Hydrochloride (vgl. hierzu A. Pinner, «Die Imidoäther und ihre Derivate» [Berlin 1892], S. 53) und anschliessende Umsetzung mit den für die Synthese der gewünschten Produkte benötigten Aminen (analog F.L. Pyman, J. Chem. Soc. 1923, 3359, A. Pinner, «Die Imidoäther und ihre Derivate» [Berlin 1892], S. 152).

Die nach dem neuen Verfahren hergestellten Imidazolessigsäurederivate der allgemeinen Formel (I) stellen Zwischenprodukte für wertvolle Arzneimittel (z.B. Entzündungshemmer: 5-Imidazolessigsäurederivate, wie sie in JP 50084567 beschrieben werden, besitzen antipyretische Eigenschaften) und Pflanzen-Schutzmittel dar.

Beispiel 1
4(5)-Äthoxycarbonylmethyl-imidazol

Zu einer Lösung von 8,25 g 4-Chloracetessigsäureäthylester in 250 ml Acetonitril werden 19 ml Trimethylchlorsilan und 42 ml Hexamethyldisilazan gegeben. Die Reaktionsmischung wird eine Stunde unter Rückfluss erhitzt und dann portionsweise langsam während ca. 14 Stunden mit insgesamt 31,2 g Formamidinacetat versetzt. Man lässt abkühlen, gibt ein Gemisch von 1 Liter Äther/0,5 l Hexan hinzu und extrahiert 5 mal mit je 50 ml Wasser. Die wässrige Phase wird einmal mit einem Äther/Hexan (1/1)-Gemisch gewaschen, filtriert und im Vakuum zur Trockne eingeengt. Der Rückstand wird durch Säulenchromatographie an Kieselgel mit Hexan/25–100% Essigester und Essigester/0–20% Äthanol als Fliessmittel gereinigt. Man erhält 6,6 g (85%) des gewünschten Imidazolessigesters.

NMR (DMSO) 1,20 (t); 3,55; 4,05 (q); 6,85 (s); 7,45 (s)
IR (Film) 1735/cm.

Beispiel 2
4(5)-Äthoxycarbonylmethyl-imidazol

Ersetzt man den trimethylsilylierten 4-Chloracetessigsäureäthylester in Beispiel 1 durch 3-Äthoxy-4-chlorcrotonsäureäthylester (R. Findings, G. Zimmermann, U. Schmidt, Mh. Chem., 102, 214 [1971]; Ausbeute: 20%), so erhält man 0,89 g (11%) 4(5)-Äthoxycarbonyl-methyl-imidazol.

Beispiel 3
1,2-Diphenyl-4-äthoxycarbonylmethyl-imidazol

Ein Gemisch aus 9,8 g Phenylbenzamidin [die Darstellung erfolgt nach Org. Synth. Coll. Vol. IV, 769 (1963)], 8,25 g 4-Chloracetessigsäureäthylester, 100 ml Hexamethyldisilazan und 7 ml Triäthylamin wird über Nacht unter Rückfluss erhitzt. Danach wird mit 400 ml Essigester verdünnt und 5mal mit gesättigter Natriumbicarbonatlösung sowie Wasser gewaschen. Nach Extraktion der wässrigen Phasen mit Methylenchlorid werden die vereinigten organischen Phasen getrocknet und im Vakuum eingeengt. Der Rückstand wird durch Säulenchromatographie an Kieselgel mit Methylenchlorid/0,2–1% Isopropanol sowie Toluol/40% Essigester als Fliessmittel gereinigt.

Man erhält 9,1 g (59%) der Titelverbindung.
NMR ($CHCl_3$) 1,28 (t); 3,75 (d); 4,2 (q); 7,1–7,4
IR (Film) 1735/cm.

Beispiel 4
2-(4-Brom)-phenyl-4-äthoxycarbonylmethylimidazol

Ein Gemisch aus 5,88 g 4-Brombenzamidin-Hydrochlorid (die Darstellung erfolgt nach C.H. Andrews, H. King und J. Walker, Proc. roy. Soc. [B] [London] 133, 20, (1946)), 4,12 g 4-Chloracetessigsäureäthylester, 10,5 ml Hexamethyldisilazan und 3,5 ml Triäthylamin wird über Nacht unter Rückfluss erhitzt. Anschliessend wird mit 500 ml Essigester verdünnt und 3 mal mit je 100 ml gesättigter Natriumbicarbonatlösung sowie 3 mal mit je 100 ml Wasser gewaschen. Nach Extraktion der wässrigen Phasen mit Methylenchlorid werden die vereinigten organischen Phasen über Natriumsulfat getrocknet und im Vakuum zur Trockne eingeengt. Der Rückstand wird durch Säulenchromatographie an Kieselgel mit Methylenchlorid/0,1–6% Isopropanol als Fliessmittel gereinigt. Es werden 3,8 g (49,9%) der Titelverbindung erhalten.
Fp.: 165–166 °C (Toluol/Essigester).

Beispiel 5
1-Methyl-2-phenyl-4-äthoxycarbonylmethyl-imidazol

Ein Gemisch aus 6,12 g N-Methyl-benzamidin-Hydrochlorid, 4,95 g 4-Chloracetessigsäureäthylester, 18,5 ml Hexamethyldisilazan, 1,27 ml Trimethylchlorsilan und 350 ml Acetonitril wird unter Rückfluss so lange erhitzt, bis die analytische DC-Kontrolle kein Ausgangsprodukt mehr anzeigt (ca. 72 Stunden). Man lässt abkühlen, verringert das Volumen der Reaktionslösung durch Einengen im Vakuum um etwa die Hälfte, versetzt dann mit 200 ml Wasser und extrahiert mehrmals mit einem Äther/Hexan-(1/1)-Gemisch. Die vereinigten organischen Phasen werden über Magnesiumsulfat getrocknet, das Lösungsmittel im Vakuum entfernt und der Rückstand durch Säulenchromatographie an Kieselgel mit Pentan/0–100% Essigester als Fliessmittel gereinigt.

Man erhält 3,53 g (48%) der Titelverbindung.
IR (Film) 1735/cm
NMR (DMSO) 1,20 (t); 3,55; 3,72; 4,10 (q); 7,10; 7,3–7,7

Als polares Nebenprodukt wird das 1,5-Stellungs-Isomere (1-Methyl-2-phenyl-5-äthoxycarbonylmethyl-imidazol) in geringer Menge isoliert.

Beispiel 6
1-Methyl-2-(3-chlor)-phenyl-4-äthoxycarbonylmethyl-imidazol

10,25 g N-Methyl-3-chlorbenzamidin-Hydrochlorid werden zunächst 9 Stunden zusammen mit 8,25 g 4-Chloracetessigsäureäthylester, 21 ml Hexamethyldisilazan, 6,4 ml Trimethylchlorsilan und 250 ml Acetonitril unter Rückfluss erhitzt, danach wird, um die Reaktionszeit abzukürzen, bei 110 °C Badtemperatur das Lösungsmittel abdestilliert, die Reaktionslösung mit 50 ml Hexamethyldisilazan versetzt und eine Stunde bei 140 °C Badtemperatur gehalten. Man lässt abkühlen, versetzt mit Wasser und extrahiert mehrmals mit einem Äther/Hexan (1/1)-Gemisch. Die vereinigten organischen Phasen werden über Magnesiumsulfat getrocknet, im Vakuum eingeengt und der Rückstand durch Säulenchromatographie an Kieselgel mit Hexan/10–100% Essigester bzw. Methylenchlorid/10% Isopropanol als Fliessmittel gereinigt.

Man erhält 4,6 g (33%) der Titelverbindung.
IR (Film) 1735/cm
NMR ($CHCl_3$) 1,30 (t); 3,70; 3,73; 4,20 (q); 6,95; 7,3–7,7.

Die Ausgangsverbindung wird wie folgt hergestellt:

6a) 3-Chlor-benzimidoäthylester-Hydrochlorid

Eine Lösung von 50 g 3-Chlorbenzonitril in Äthanol und Äther wird mit trockenem Chlorwasserstoff gesättigt und anschliessend bei 3 °C stehengelassen. Aus der Reaktionslösung kristallisieren 70 g 3-Chlor-benzimidoäthylester-Hydrochlorid aus.

6b) N-Methyl-3-chlorbenzamidin-Hydrochlorid

Zu einer aus 4,9 g Natrium in 86 ml absolutem Äthanol hergestellten Natriumäthylat-Lösung werden 14,2 g Methylammoniumchlorid gegeben. Nach einer Stunde Rühren wird diese Suspension zu 35 g 3-Chlorbenzimidoäthylester-Hydrochlorid gegeben und über Nacht bei 3 °C aufbewahrt. Nach Filtration wird die Lösung zur Trockne eingeengt und der Rückstand in Wasser gelöst. Die gewünschte Substanz wird durch Fällung mit Aceton/Äther erhalten. Es werden 24,8 g vom Schmelzpunkt 209 °C isoliert.

Beispiel 7
4-(5)-Methoxycarbonylmethyl-2-phenyl-imidazol

Ein Gemisch aus 109 Benzamidin-Hydrochlorid, 7,55 g 4-Chloracetessigsäuremethylester, 33 ml Hexamethyldisilazan, 6,3 ml Trimethylchlorsilan und 300 ml Acetonitril wurde mehrere Stunden unter Rückfluss gekocht, bis die analytische DC-Kontrolle kein Ausgangsmaterial mehr anzeigt. Danach wird das Reaktionsgemisch auf 500 ml Wasser gegossen und 5 mal mit je 100 ml Methylenchlorid extrahiert. Der Inhaltsstoff der organischen Phase wird durch Säulenchromatographie an Kieselgel mit Hexan/50–100% Essigester als Fliessmittel gereinigt. Nach Umkristallisation aus Benzol erhält man 6,5 g des gewünschten Imidazols mit einem Schmelzpunkt von 122–124 °C.
IR (Film) 1730/cm
NMR (DMSO) 3,65; 7,05; 7,3–7,9.

Beispiel 8
2(2-Pyridyl)-imidazol-4(5)-essigsäuremethylester

4,73 g (0,03 Mol) 2-Pyridylamidin-hydrochlorid (F.C. Schaefer und G.A. Peters, J. Org. Chem. 26, 412 (1961)) werden 14 Stunden mit 4,5 g (3,6 ml = 0,03 Mol) 4-Chloracetessigsäuremethylester, 12,65 ml (0,06 Mol) Hexamethyldisilazan und 14,3 ml (0,06 Mol) Tributylamin auf 140 °C unter Rühren erhitzt. Nach Abkühlen wird mit Methylenchlorid und gesättigter Natriumbikarbonatlösung aufgenommen und die wässrige Phase mehrfach mit Methylenchlorid nachextrahiert. Nach Trocknen mit $Na_2SO_4$ wurde die Methylenchloridphase abgedampft, wobei 4,7 g (72%) roher 2-Pyridyl-imidazol-4(5)-essigsäuremethyl-

ester erhalten wurde, der im Dünnschichtsystem Essigester-Methanol-Triäthylamin (75:20:5) homogen war (Rf = 0,55). Eine einfache Filtration in Essigester über 100 g Silicagel ergab 4,3 g (66%) reine Substanz.
NMR (DMSO-$D_6$): 3,65 (s), 7,05 (s), 7,3 (m), 7,9 (m), 8,55 (m).

**Patentanspruch**

Verfahren zur Herstellung von Imidazolessigsäurederivaten der allgemeinen Formel I,

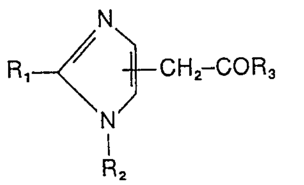 (I),

in der

$R_1$ Wasserstoff, eine geradkettige oder verzweigte Alkylgruppe mit 1–24 C-Atomen, Cyclopentyl, Cyclohexyl, Cycloheptyl, Äthylcyclopentyl, Methylcyclohexyl, einen Phenylalkylrest mit 7–10 C-Atomen, die Reste Phenyl, α-Naphthyl, β-Naphthyl und Phenanthryl, die durch Chlor, Brom, Methyl, Methoxy oder Trifluormethyl substituiert sein können, eine mono- oder bicyclische heterocyclische Gruppe mit 1–3 N-, O- oder S-Heteroatomen,

$R_2$ Wasserstoff, eine geradkettige oder verzweigte Alkylgruppe mit 1–24 C-Atomen, Cyclopentyl, Cyclohexyl, Cycloheptyl, Äthylcyclopentyl, Methylcyclohexyl, einen Phenylalkylrest mit 7–10 C-Atomen, die Reste Phenyl, α-Naphthyl, β-Naphthyl und Phenanthryl, die durch Chlor, Brom, Methyl, Methoxy oder Trifluormethyl substituiert sein können, eine mono- oder bicyclische heterocyclische Gruppe mit 1–3 N-, O- oder S-Heteroatomen,

$R_1$ und $R_2$ gemeinsam eine Alkylengruppe mit 4–7 C-Atomen und

$R_3$ eine geradkettige oder verzweigte Alkoxygruppe mit 1–10 C-Atomen oder eine Phenylalkoxygruppe mit 7–10 C-Atomen bedeuten, dadurch gekennzeichnet, dass man Amidine bzw. deren Salze der allgemeinen Formel II,

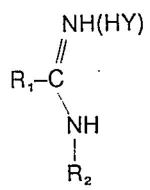 (II),

in der $R_1$ und $R_2$ die oben angegebenen Bedeutungen haben und HY anorganische Mineralsäuren oder organische Säuren darstellt, mit Acetessigsäurederivaten der allgemeinen Formel III,

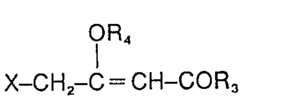 (III),

in der $R_3$ die oben angegebene Bedeutung hat und X die Halogene Fluor, Chlor, Brom und Jod und $R_4$ einen Tri-($C_1$–$C_4$)-alkylsilylrest oder einen $C_1$–$C_4$-Alkylrest darstellen, umsetzt.

**Claim**

Process for the production of imidazole acetic acid derivatives of the general formula I:

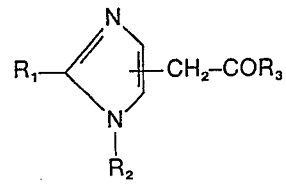 (I),

in which

$R_1$ is hydrogen, a straight or branch-chained alkyl group with 1–24 C-atoms, cyclopentyl, cyclohexyl, cycloheptyl, ethylcyclopentyl, methylcyclohexyl, a phenylalkyl group with 7–10 C-atoms, a phenyl, α-naphthyl, β-naphthyl or phenanthryl group, which group may be substituted by chlorine, bromine, methyl, methoxy or trifluoromethyl, or a mono- or bi-cyclic heterocyclic group with 1–3 N-, O- or S-heteroatoms,

$R_2$ is hydrogen, a straight or branch-chained alkyl group with 1–24 C-atoms, cyclopentyl, cyclohexyl, cycloheptyl, ethylcyclopentyl, methylcyclohexyl, a phenylalkyl group with 7–10 C-atoms, a phenyl, α-naphthyl, β-naphthyl or phenanthryl group, which group may be substituted by chlorine, bromine, methyl, methoxy or trifluoromethyl, or a mono- or bi-cyclic heterocyclic group with 1–3 N-, O- or S-heteroatoms,

$R_1$ and $R_2$ together form an alkylene group with 4–7 C-atoms, and

$R_3$ is a straight or branch-chained alkoxy group with 1–10 C-atoms or a phenylalkoxy group with 7–10 C-atoms,

characterised in that the corresponding amidine or salt of the general formula II,

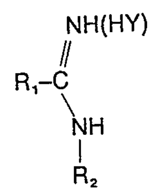 (II),

in which $R_1$ and $R_2$ are as defined above and HY is an inorganic mineral acid or an organic acid, is reacted with an acetoacetic acid derivative of the general formula III,

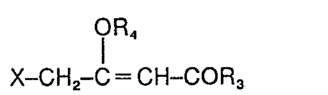 (III),

in which $R_3$ is as defined above and X is fluorine, chlorine, bromine or iodine, and $R_4$ is a tri-($C_1$–$C_4$)-alkylsilyl group or a $C_1$–$C_4$ alkyl group.

**Revendication**

Procédé pour préparer des dérivés d'acides imidazolyl-acétiques répondant à la formule générale I:

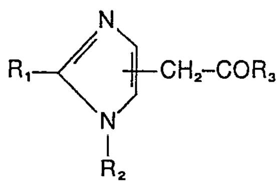

(I)

dans laquelle

$R_1$ représente l'hydrogène, un radical alkyle, linéaire ou ramifié, contenant de 1 à 24 atomes de carbone, un radical cyclopentyle, cyclohexyle, cycloheptyle, éthylcyclopentyle ou methyl-cyclohexyle, un radical phénylalkyle contenant de 7 à 10 atomes de carbone, un radical phényle, α-naphtyle, β-naphtyle ou phénanthryle éventuellement porteur d'un atome de chlore ou de brome ou d'un radical méthyle, méthoxy ou trifluorométhyle, ou un radical hétérocyclique mono- ou bicyclique qui contient de 1 à 3 hétéroatomes pris dans l'ensemble constitué par N, O et S,

$R_2$ représente l'hydrogène, un radical alkyle, linéaire ou ramifié, contenant de 1 à 24 atomes de carbone, un radical cyclopentyle, cyclohexyle, cycloheptyle, éthylcyclopentyle ou méthylcyclohexyle, un radical phénylalkyle contenant de 7 à 10 atomes de carbone, un radical phényle, α-naphtyle, β-naphtyle ou phénanthryle éventuellement porteur d'un atome de chlore ou de brome ou d'un radical méthyle, méthoxy ou trifluorométhyle, ou un radical hétérocyclique monocyclique ou bicyclique contenant de 1 à 3 hétéroatomes pris dans l'ensemble constitué par N, O et S, ou encore

$R_1$ et $R_2$ forment ensemble un radical alkylène contenant de 4 à 7 atomes de carbone, et

$R_3$ représente un radical alcoxy, linéaire ou ramifié, contenant de 1 à 10 atomes de carbone ou un radical phénylalcoxy contenant de 7 à 10 atomes de carbone,

procédé caractérisé en ce qu'on fait réagir des amidines ou leurs sels répondant à la formule générale II:

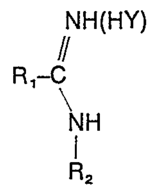

(II)

dans laquelle $R_1$ et $R_2$ ont les significations indiquées ci-dessus et HY représente un acide minéral ou organique, avec des dérivés d'acides acétylacétiques répondant à la formule générale III

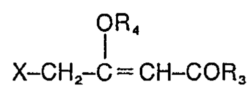

(III)

dans laquelle $R_3$ a la signification précédemment donnée et X représente un halogène, en l'espèce le fluor, le chlore, le brome ou l'iode, et $R_4$ un radical tris-($C_1$–$C_4$) alkyl-silyle ou un radical alkyle en $C_1$–$C_4$.